# FASCICULE DE BREVET EUROPEEN

(11) **EP 1 194 154 B1**
(45) Date de publication et mention de la délivrance du brevet: **09.03.2005**
(21) Numéro de dépôt: 00953259.9
(22) Date de dépôt: 19.07.2000
(51) Int. Cl.: A61K 35/74, A23L 1/308, A61P 1/00, A61P 31/00

(54) **UTILISATION DE BACTERIES PROPIONIQUES POUR LA PRODUCTION D'ACIDE PROPIONIQUE ET/OU DE PROPIONATES DANS LE COLON**
VERWENDUNG VON PROPIONÄURE BAKTERIEN FÜR DIE HERSTELLUNG VON PROPIONSÄURE IM DICKDARM
USE OF PROPIONIC BACTERIA FOR PRODUCING PROPIONIC ACID AND/OR PROPIONATES IN THE COLON

(30) Priorité: 20.07.1999 FR 9909385
(43) Date de publication de la demande: 10.04.2002
(73) Titulaire: Laboratoires Standa S.A., 14050 Caen Cédex (FR)
(72) Inventeur: ROUSSEL, Edmond, Daniel, F-14210 Avenay (FR); LEGRAND, Charles, Gabriel, 14000 Caen (FR); LEGRAND, Marc, Henri, 14000 Caen (FR); ROLAND, Nathalie, 35000 Rennes (FR); BOUGLE, Dominique, F-14000 Caen (FR)
(74) Mandataire: Cabinet HERRBURGER
(86) Numéro de dépôt international: PCT/FR2000/002072
(87) Numéro de publication internationale: WO 2001/005413

(56) Documents cités:
- WO-A-98/27991
- FR-A- 2 741 509
- KANEKO T ET AL: "GROWTH STIMULATOR FOR FIFIDOBACTERIA PRODUCED BY PROPIONIBACTERIUM FREUDENREICHII AND SEVERAL INTESTINAL BACTERIA" JOURNAL OF DAIRY SCIENCE,US,AMERICAN DAIR SCIENCE ASSOCIATION. CHAPAIGN, ILLINOIS, vol. 77, no. 2, 1 janvier 1994 (1994-01-01), pages 393-404, XP000590802 ISSN: 0022-0302
- DATABASE CHEMABS [en ligne] CHEMICAL ABSTRACTS SERVICE, COLUMBUS, OHIO, US; MANTERE-ALHONEN, S.: "Propionibacteria used as probiotics - a review" retrieved from STN Database accession no. 124:143825 HCA XP002139247 & LAIT (1995), 75(4-5), 447-52,
- DATABASE FSTA [en ligne] INTERNATIONAL FOOD INFORMATION SERVICE (IFIS), FRANFURT/MAIN, DE; CRESCI A ET AL: "The effect of sucrose or starch-based diet on short-chain fatty acids and faecal microflora in rats." Database accession no. 1999-00-a0963 XP002139248 & JOURNAL OF APPLIED MICROBIOLOGY, 86,(2) 245-250 1999

## Description

La présente invention se rapporte à l'utilisation de bactéries propioniques en vue d'optimiser la production d'acide propionique et/ou de propionates et le cas échéant d'acide acétique et/ou d'acétates au niveau du côlon.

Depuis quelques années, les spécialistes nutritionnistes conseillent à leurs patients une alimentation riche en fibres auxquelles ils attribuent des effets physiologiques et métaboliques pouvant bénéficier à la santé.

Il est connu que les fibres alimentaires résistent à la digestion enzymatique dans l'intestin grêle et ne sont dégradées et assimilées qu'au niveau du côlon, c'est-à-dire de la partie terminale de l'intestin. L'effet bénéfique susmentionné ne peut donc s'exercer qu'à la condition que cette dégradation et cette assimilation soient aussi complètes que possibles à cet endroit préterminal : le côlon.

Or, on a pu établir que ces réactions biologiques sont consécutives à la fermentation anaérobie des fibres alimentaires sous l'action de micro-organismes dans le côlon. Cette fermentation aboutit à la production d'acides gras à chaîne courte (AGCC), d'hydrogène, de dioxyde de carbone et de biomasse.

Ces acides gras à chaîne courte sont essentiellement l'acide acétique, l'acide propionique et l'acide butyrique ; dans l'organisme sain, ils ne peuvent être produits qu'au niveau du côlon, vu qu'il s'agit là du seul endroit du corps humain où règnent les conditions anaérobies strictes permettant la fermentation à la base de leur synthèse, à l'exception de l'acide acétique dont une très faible quantité peut être produite au niveau hépatique.

Or, différentes études ont prouvé l'importance de ces acides gras à chaîne courte qui sont bénéfiques à la santé.

D'après la littérature, il semblerait que les rôles physiologiques de ces trois acides gras à chaîne courte seraient différents les uns des autres : en effet, les acides acétiques et propioniques seraient directement conduits vers le foie où la totalité de l'acide propionique serait métabolisée alors qu'une partie de l'acide acétique serait ensuite conduite à différents tissus, tandis que l'acide butyrique serait utilisé plus spécifiquement au niveau interne de la paroi colique.

La synthèse des acides gras à chaîne courte implique donc la présence dans le côlon, d'une part, d'un substrat à base de fibres facile à apporter par la nourriture et, d'autre part, d'une flore bactérienne équilibrée et adaptée, présente de façon optimum et constante.

Cette flore bactérienne peut provenir soit de la flore endogène persistante de chaque individu, soit de l'alimentation.

Il est, en effet, bien connu que le contenu du tube digestif humain qui est spécifique à chaque individu et correspond approximativement à 1 à 1,5 kg de matières alimentaires en cours de transformation digestive, renferme une importante population de micro-organismes constituée par un mélange de nombreuses espèces pouvant être évaluée à 10¹¹ à 10¹² cellules par gramme dans le côlon ; cette population constitue une masse bactérienne d'un poids certain dont l'équilibre bon ou mauvais ne peut que difficilement être modifié radicalement et surtout durablement par le seul fait de l'alimentation courante.

Par ailleurs, l'alimentation que l'on ingère journellement n'est jamais stérile et est donc plus ou moins chargée de bactéries (lait, produits laitiers fermentés, fromage, cidre, vin, bière, charcuterie, etc.). Les modifications de la flore colique consécutives à l'absorption de ces bactéries ne peuvent toutefois être que temporaires.

Il est, en outre, à noter qu'il a déjà été proposé de tenter de modifier la population microbienne du tractus intestinal par l'administration et en particulier l'ingestion volontaire de cellules bactériennes réputées bénéfiques à la santé (dites probiotiques) en particulier de bactéries lactiques ou de bactéries bifides.

L'introduction dans l'organisme d'une population importante de ces bactéries soit par le biais d'une alimentation particulière, soit par l'ingestion directe de ces cellules microbiennes, a plus particulièrement été proposée dans le but de limiter le développement des espèces pathogènes et putréfiantes : il est en effet connu que la flore endogène présente dans le côlon est répartie en différents groupes bactériens dont certains sont inoffensifs, voire bénéfiques, tandis que d'autres, en particulier des clostridium et des putréfiants conduisent à la production de substances toxiques et influencent négativement la santé.

L'idée à la base de l'invention a consisté à introduire régulièrement dans l'organisme, par voie buccale, une quantité importante d'une flore microbienne probiotique apte à favoriser la synthèse régulière d'acides gras à chaîne courte au niveau colique.

Parmi les espèces microbiennes pouvant être mises en oeuvre à cet effet, les bactéries lactiques ne sont que peu adaptées puisque par leur nature, elles produisent surtout et avant tout de l'acide lactique et très secondairement un peu d'acide acétique mais pas d'acide propionique ni d'acide butyrique.

En revanche, des bactéries d'un autre type, les bactéries propioniques, sont capables de produire abondamment de l'acide propionique et de l'acide acétique donc, les deux acides gras à chaîne courte qui sont appelés à irriguer les réseaux tissulaires, ce par exemple selon un pourcentage de 2/3 d'acide propionique pour 1/3 d'acide acétique. Ces bactéries sont présentes en alimentation humaine depuis des siècles, en particulier dans les fromages à pâte pressée cuite ; de plus, elles présentent l'avantage d'être mieux armées que les bactéries lactiques pour avoir une activité dans le côlon où l'anaérobiose est totale, et par ailleurs, d'être plus résistantes aux stress technologiques que les bactéries lactiques et les bactéries bifides.

Il est à noter qu'il a déjà été proposé dans la littérature de faire absorber des bactéries propioniques, en particulier pour stimuler le développement des bactéries bifides dans l'intestin (document WO-97/19689) ou encore pour dégager du monoxyde d'azote dans le tube digestif humain ou animal (document WO-98/27991). On n'avait cependant jusqu'à présent jamais eu l'idée d'utiliser ces bactéries pour la production d'acides gras à chaîne courte au niveau du côlon.

La présente invention concerne donc l'utilisation de bactéries propioniques sélectionnées en fonction de leur caractère peu autolytique et de leur aptitude à résister aux sels biliaires pour l'obtention d'une composition d'alimentation courante ou d'une composition diététique ou médicamenteuse absorbable par l'homme ou l'animal, élaborée pour que les bactéries soient protégées au moins partiellement vis-à-vis de l'acidité gastrique, renfermant au moins 10⁶ cellules/gramme de ces bactéries, susceptible de stimuler et d'augmenter de façon significative la synthèse d'acide propionique et/ou de propionate et le cas échéant d'acide acétique et/ou d'acétate au niveau du côlon par fermentation bactérienne anaérobie.

Pour que ces bactéries puissent avoir l'effet bénéfique escompté, il est indispensable de choisir des souches peu autolytiques aptes à atteindre, sans dommage, le côlon, éventuellement à s'y développer et à produire des quantités suffisantes d'acide propionique.

Il est bien connu que les deux stress principaux, auxquels les bactéries ingérées sont soumises lors de leur passage dans la partie haute du tube digestif, sont liés, d'une part, à l'acidité du milieu stomacal (pH 4 à 1) et, d'autre part, à la présence de sels biliaires dans l'intestin grêle (de l'ordre de 15 mmol/l au maximum au niveau du duodénum).

Or, on a pu établir que des bactéries ayant été exposées à l'acidité stomacale sont fragilisées et par suite inaptes à résister aux sels biliaires, ce même si elles demeurent viables à la sortie de l'estomac.

Par suite, conformément à l'invention, il est indispensable de faire subir aux bactéries propioniques un traitement de nature à leur permettre de ne pas subir le stress gastrique correspondant en règle générale à une encapsulation qui peut être volontaire ou involontaire par exemple dans le cas d'un aliment de type fromage.

Cette situation a été mise en lumière grâce à un test par lequel on a évalué l'influence du pH acide et des sels biliaires successivement ou individuellement sur la viabilité de deux souches de bactéries propioniques laitières appartenant à la collection TL du LRTL (Laboratoire de Recherches de Technologie laitière - INRA de Rennes), à savoir les souches TL 162 et TL 24 appartenant à l'espèce P.*freudenreichii subsp shermanii.*

Les résultats de ce test sont décrits ci-dessous.

Les bactéries ont été cultivées à 30°C sur milieu YEL pendant 2 jours (début de phase stationnaire). La densité optique à 650 nm était respectivement de 2,28 et 2,64 pour TL 162 et TL 24.

### - Stress acide

Les cultures ont été diluées au 1/10^{ème} dans du milieu S (tryptone-lactate) à pH 2,5 (pH final de 3,0). Après une incubation à 37°C pendant 45 min, les cultures ont été centrifugées et les bactéries reprises dans le même volume de YEL. Des numérations ont été faites avant et à la fin de l'incubation, et la reprise de la croissance a été suivie par des mesures de DO pendant 5 jours à 37°C.

### - Stress biliaire

Les cultures initiales ont été centrifugées et les bactéries reprises dans un volume 10 fois supérieur de YEL contenant 0,3 % de bile de boeuf (à ~50 % de sels biliaires). Après une incubation à 37°C pendant 90 min, les cultures ont été centrifugées et les bactéries reprises dans le même volume de YEL. Des numérations ont été faites avant et à la fin de l'incubation, et la reprise de la croissance a été suivie par des mesures de DO pendant 5 jours à 37°C.

### - Stress acide et biliaire successifs

Les bactéries ont subi un stress acide comme décrit précédemment, mais après centrifugation, les cellules ont été reprises dans du YEL contenant 0,3 % de bile. Après une deuxième incubation à 37°C pendant 90 min, les cultures ont été centrifugées et les bactéries reprises dans le même volume de YEL. Des numérations ont été faites avant et à la fin des incubations et la reprise de la croissance a été suivie par des mesures de DO pendant 5 jours à 37°C.

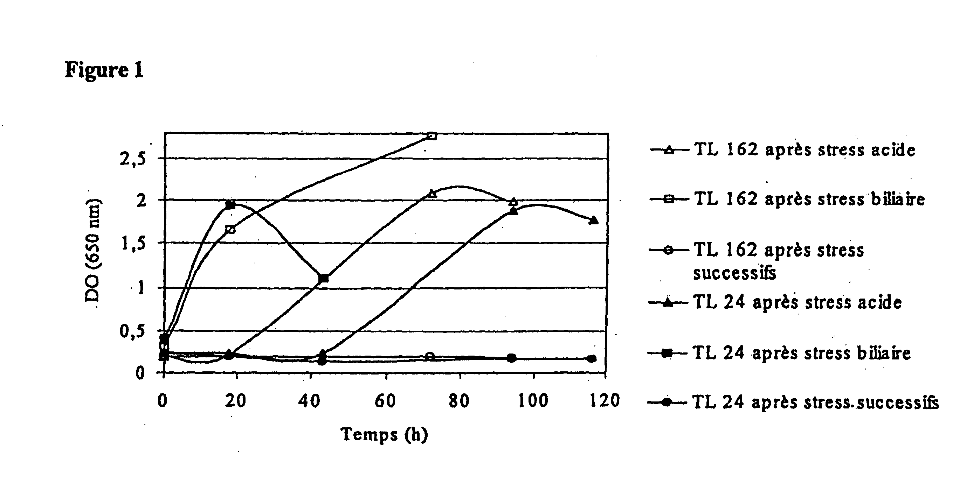

Les résultats obtenus sont rapportés, d'une part, sur le tableau 1 ci-dessous qui mentionne l'influence des stress acide et/ou biliaire sur la viabilité des bactéries et, d'autre part, sur la figure 1 qui est un schéma représentant la reprise de la croissance après les différents stress.

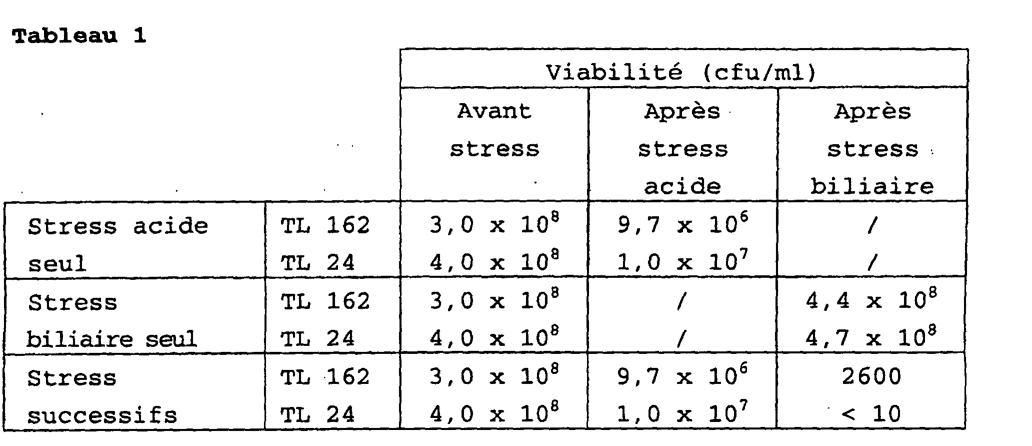

On a ainsi pu établir que :
- l'acidité entraîne une mortalité importante des bactéries (96,8 % pour TL 162 et 97,5 % pour TL 24), ce qui explique un délai assez long pour la reprise de la croissance (Figure 1) .
- la bile n'entraîne aucune mortalité des bactéries, d'où une reprise très rapide de la croissance.
- lorsque l'on soumet les bactéries à la bile, après un stress acide préalable, cela conduit à une mortalité quasi totale des bactéries. Ce résultat, totalement inattendu, indique donc que des bactéries, qui ont subi un stress acide et qui toutefois restent viables, deviennent totalement sensibles à la bile alors que sans stress acide préalable, ces mêmes bactéries sont totalement résistantes aux sels biliaires.

Tenant compte de cette situation, on a effectué des essais de préadaptation dans le but d'augmenter la résistance des bactéries. On sait en effet qu'un pré-stress acide (pH 4,5-5) protège efficacement les cellules contre un stress acide (pH 2).

On a ainsi effectué trois essais d'adaptation sur TL 162 :
- *pré-stress acide* : incubation préliminaire des cellules à 37°C pendant 30 min à pH 5
- *pré-stress biliaire* : incubation pendant 30 min en présence de 0,08 % de bile
- *pré-stress acide et biliaire :* incubation pendant 30 min à pH 5 et en présence de 0,08 % de bile.

On a appliqué le même protocole que précédemment et obtenu les résultats rapportés dans le tableau 2 ci-dessous :

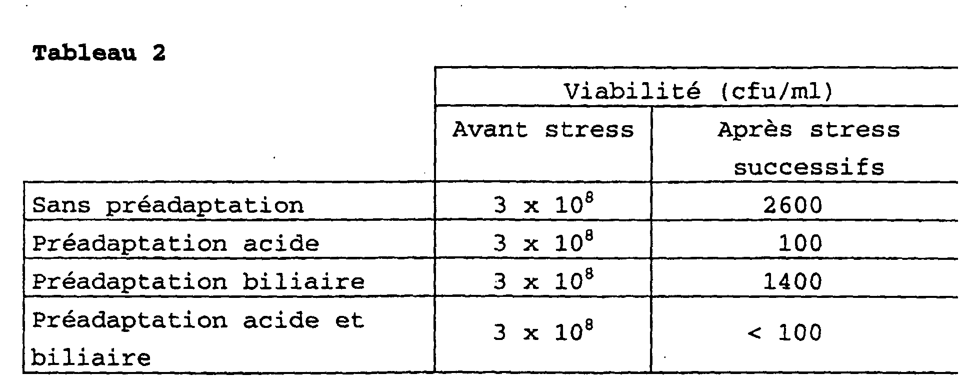

On a ainsi pu établir qu'une préadaptation acide fragilise encore plus les cellules, alors qu'une préadaptation biliaire est sans effet.

Ces résultats ont donc permis de mettre en évidence la nécessité de traiter les stress successivement, et non pas séparément comme décrit dans la plupart des études qui ont été réalisées dans ce domaine.

On peut cependant supposer qu'in vivo les conditions sont moins drastiques pour les bactéries (effet tampon de l'aliment dans l'estomac, effet bactéricide moindre des sels biliaires sous forme micellaire avec les phospholipides).

Compte tenu de ce qui précède, pour augmenter la quantité de bactéries viables, une amélioration de leur résistance au pH acide n'est pas efficace puisque les bactéries restent sensibles à l'effet des sels biliaires.

En revanche, en protégeant les bactéries du stress acide, en particulier en les ingérant préconditionnées dans des gélules gastrorésistantes, on peut retrouver dans les fèces des bactéries naturellement résistantes à la bile, ce à un niveau élevé de viabilité.

Compte tenu de ces résultats, on a effectué un test complémentaire pour comparer l'aptitude de différentes souches de bactéries propioniques à produire des quantités importantes d'acide propionique après avoir été mises en contact avec des sels biliaires.

Dans ce test, on a comparé 33 souches de bactéries propioniques laitières appartenant à la collection TL du LRTL (INRA de Rennes) selon leur aptitude à survivre en présence de bile et à produire ensuite de l'acide propionique :
- 20 souches appartenant à l'espèce P. *freudenreichii subsp shermanii*
- 6 souches appartenant à l'espèce P. *freudenreichii subsp freudenreichii*
- 7 souches appartenant à l'espèce P. *acidipropionici*

Le protocole opérationnel a été le suivant :
Des cultures en début de phase stationnaire (2 à 3 jours de culture en milieu YEL incubé à 30°C) ont été diluées au 1/10^{ème} dans du milieu YEL contenant 0,6 % de bile de boeuf (environ 7-8 mmol/l de sels biliaires). Cette concentration en bile a été choisie de façon à discriminer au mieux les souches entre elles et constitue des teneurs en sels biliaires du même ordre que celles rencontrées dans le duodénum.

Les dilutions ont été incubées à 37°C pendant 90 min, puis centrifugées. Les bactéries ont été reprises dans du milieu YEL (volume initial) et remises à incuber à 37°C.

Après 24 h d'incubation, la DO à 650 nm a été mesurée pour évaluer la reprise de la croissance. Le surnageant a été récolté puis congelé pour doser les acides gras.

Pour certaines souches, l'expérience a été renouvelée de façon à confirmer les résultats.

Les valeurs des densités optiques à 650 nm avant le stress biliaire et 24 h après la fin du stress sont rapportées dans le tableau 3 ci-dessous :

**Tableau 3**

| Souches : | DO avant le stress biliaire (DO initiale/10) | DO à 24 h d'incubation après le stress biliaire | % de DO à 24 h/DO initiale |
|---|---|---|---|
| *P. shermanii* TL 125 | 0,32 | 2,02 | 63 |
| TL 134 | 0,29 - 0,26 | 2,60 - 2,04 | 90 - 78 |
| TL 144 | 0,39 | 3,62 | 93 |
| TL 146 | 0,27 | 1,56 | 58 |
| TL 147 | 0,28 | 1,23 | 44 |
| TL 148 | 0,23 | 0,04 | 2 |
| TL 160 | 0,36 - 0,32 | 4,67 - 4,03 | 130 - 126 |
| TL 167 | 0,26 | 1,05 | 40 |
| TL 168 | 0,32 | 0,57 | 18 |
| TL 4 | 0,32 | 0,29 | 9 |
| TL 14 | 0,23 | 0,73 | 32 |
| TL 17 | 0,29 | 0,55 | 19 |
| TL 22 | 0,28 | 1,39 | 50 |
| TL 24 | 0,28 | 1,65 | 59 |
| TL 162 | 0,20 | 2,08 | 104 |
| TL 34 | 0,26 - 0,27 | 3,40 - 3,87 | 131 - 143 |
| TL 50 | 0,26 | 2,05 | 79 |
| TL 61 | 0,23 | 1,31 | 57 |
| TL 63 | 0,27 - 0,26 | 3,26 - 3,55 | 121 - 137 |
| TL 40 | 0,30 | 1,46 | 49 |
| *P. freundenreichi* TL 142 | 0,34 - 0,31 | 2,95 - 2,80 | 87 - 90 |
| TL 3 | 0,24 | 2,66 | 111 |
| TL 19 | 0,30 | 2,66 | 89 |
| TL 37 | 0,23 | 1,79 | 78 |
| TL 33 | 0,26 | 2,38 | 92 |
| TL 64 | 0,29 | 0,31 | 11 |
| P. *acidipropionici* TL 2 | 0,38 | 2,30 | 61 |
| TL 9 | 0,34 | 2,29 | 67 |
| TL 15 | 0,34 | 3,09 | 91 |
| TL 54 | 0,34 | 1,39 | 41 |
| TL 47 | 0,20 | 0,22 | 11 |
| TL 223 | 0,29 - 0,38 | 2,59 - 2,91 | 89 - 77 |
| TL 249 | 0,44 | 3,40 | 77 |

Il est à noter que pour certaines souches, la DO finale est supérieure à la DO initiale, ce qui peut s'expliquer par la croissance reprise à 37°C et non à 30°C comme initialement.

En fonction des résultats obtenus, on peut schématiquement distinguer trois groupes de souches :
- les souches se distinguant par une reprise rapide de la croissance, indiquant une faible mortalité des cellules due à la bile (parmi elles TL 34, TL 160, TL 63, TL 33, TL 15, TL 3, TL 162),
- des souches très peu résistantes à la bile, présentant une reprise de la croissance très faible ou nulle (TL 148, TL 4, TL 64, TL 47),
- des souches intermédiaires, caractérisées par une mortalité modérée due à la bile (TL 146, TL 147, TL 167, TL 168, TL 14, TL 17, TL 22, TL 24, TL 61, TL 40, TL 54).

Seules les souches possédant un ratio [(DO à 24 h/DO initiale) x 100] supérieur à 60 % (seuil choisi arbitrairement) ont été sélectionnées pour la mesure du lactate, de l'acétate et du propionate par HPLC dans les surnageants congelés. La teneur initiale du milieu YEL en lactate était de 11,4 g/l.

Les concentrations en lactate, acétate et propionate des surnageants récupérés après 24 h d'incubation, sont rassemblées dans le tableau 4 ci-dessous.

**Tableau 4**

| Souches : | Lactate consommé | Acétate produit | Propionate produit |
|---|---|---|---|
| | (en g/l) | | |
| *P . shermanii* TL 125 | 5,6 | 0,6 | 2,4 |
| TL 134 | 8,9 - 7,3 | 0,9 - 0,9 | 4,0 - 3,4 |
| TL 144 | 8,8 | 1,1 | 4,6 |
| TL 160 | 9,9 - 8,8 | 1,3 - 1,1 | 4,7 - 4,6 |
| TL 162 | 5,3 | 0,6 | 2,3 |
| TL 34 | 9,7 - 10,1 | 1,0 - 1,2 | 4,9 - 4,7 |
| TL 50 | 6,6 | 0,6 | 3,7 |
| TL 63 | 9,1 - 10,0 | 1,0 - 1,1 | 4,4 - 4,4 |
| *P. freundenreichi* TL 142 | 8,1 - 8,2 | 0,9 - 0,9 | 4,5 - 4,0 |
| TL 3 | 7,8 | 0,9 | 3,6 |
| TL 19 | 6,6 | 0,7 | 3,7 |
| TL 37 | 5,5 | 0,6 | 2,5 |
| TL 33 | 7,2 | 0,8 | 3,4 |
| *P. acidipropionici* TL 2 | 2,7 | 0,4 | 1,5 |
| TL 9 | 5,3 | 0,6 | 2,4 |
| TL 15 | 3,7 | 0,4 | 2,0 |
| TL 223 | 2,8 - 3,8 | 0,4 - 0,4 | 1,8 - 1,7 |
| TL 249 | 5,2 | 0,6 | 3,2 |

Ce tableau montre que de manière prévisible, la quantité de propionate produit est corrélée avec le degré d'utilisation du lactate.

D'une façon générale, les souches appartenant à l'espèce P.acidipropionici produisent moins d'acide propionique que les souches de l'espèce P. *freundenreichii* en 24 h.

Au vu des résultats rapportés ci-dessus, certaines souches s'avèrent être de meilleurs candidats en matière de production de propionate après l'action de la bile. Il s'agit des souches produisant au moins 2 g/l de propionate dans les conditions décrites ci-dessus :
TL 134, TL 50, TL 3, TL 19, TL 33, TL 249,
et de préférence plus de 4 g/l de propionate :
TL 160, TL 144, TL 34, TL 63, TL 142.

Une expérimentation a par ailleurs été réalisée sur des volontaires sains, dans le but de vérifier l'influence bénéfique des gélules gastro-résistantes pour améliorer la survie dans l'intestin d'une souche de bactérie propionique fromagère ingérée sous forme lyophilisée (TL 162).

L'expérience a été réalisée sur 7 individus au total, avec 3 périodes de traitement de 4 semaines, séparées par 3 semaines d'intervalle.

Le traitement 1 consistait à ingérer pendant 2 semaines 5 x 10⁹ cfu/j de bactéries, conditionnées en gélules non gastrorésistantes.

Le traitement 2 consistait à ingérer pendant 2 semaines 5 x 10¹⁰ cfu/j de bactéries, conditionnées en gélules non gastrorésistantes.

Le traitement 3 consistait à ingérer pendant 2 semaines 5 x 10⁹ cfu/j de bactéries, conditionnées en gélules gastro-résistantes.

Pour chaque traitement, 4 prélèvements de fèces ont été réalisés pour rechercher les bactéries propioniques à l'aide d'un milieu sélectif (Palpropiobac®, Standa-Industrie, additionné de 4 mg/l de métronidazole). Les dates de prélèvements étaient :
- S1 . juste avant la période d'ingestion,
- S2 : 1 semaine après le début de l'ingestion,
- S3 : 2 semaines après le début de l'ingestion,
- S4 : 1 semaine après la fin de la période d'ingestion,
- HP (pour la période 3) : 3 semaines après la fin de la période d'ingestion.

Pendant toute l'expérience, les volontaires ne pouvaient pas consommer de fromage contenant des bactéries propioniques en quantité importante (Emmental, Comté, Leerdammer, Gruyères Suisses, ...), à l'exception des fromages fondus.

Le tableau 5 indique les résultats de viabilité des bactéries propioniques dans les fèces.

Avec des gélules classiques, la dose de 5 x 10⁹ cfu/j (Période 1) apparaît insuffisante pour retrouver des bactéries propioniques viables en quantité importante chez tous les volontaires. Par contre, avec la dose de 5 x 10¹⁰ cfu/j (Période 2), tous les volontaires hébergent plus de 5 log cfu/g de bactéries propioniques viables dans les fèces, dès la première semaine de traitement. Toutefois, les niveaux maximum de viabilité observés avec les doses ne sont pas différentes (-7 log).

L'utilisation de gélules gastro-résistantes (Période 3) améliore la viabilité des bactéries propioniques dans les fèces, notamment chez les volontaires chez qui on en retrouvait peu lors du 1^{er} traitement (vol. 1, 2 et 6). En fait, par rapport à la Période 2, les niveaux de viabilité obtenus sont en moyenne équivalents.
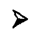 sur la base de 5 x 10⁹ cfu/j, l'utilisation des gélules gastro-résistantes se justifie donc pour un certain nombre d'individus (vol. 1, 2, 6), pour les autres elles n'améliorent pas ou peu la viabilité (vol. 3, 4 et 5),
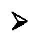 elles apportent des résultats à peu près équivalents à des gélules classiques contenant 5 x 10¹⁰ cfu.

Les AGCC ont été mesurés dans les fèces par chromatographie en phase gazeuse. Les quantités de propionate dans les fèces sont indiquées dans le tableau 6. Pour l'analyse statistique, 2 groupes de valeurs ont été comparées 2 à 2 : les valeurs correspondantes aux échantillons de fèces où les bactéries propioniques n'étaient pas détectées (< 4 log), pour tous traitements et périodes confondus et les valeurs correspondantes aux échantillons de fèces où les bactéries propioniques ont été numérées à plus de 6 log cfu/g. Dans le premier cas, la quantité moyenne de propionate est de 5,06 ± 2,56 µmol/g (n = 25) et dans le second cas, elle est de 7,19 ± 3,18 µmol/g (n = 30). Ces 2 valeurs sont significativement différentes à p < 0,02 (test de Student). Pour les autres AGCC, il n'y a pas de différences significatives. Cette expérience montre donc que la présence de quantités importantes (> 6 log cfu/g) de bactéries propioniques dans le côlon consécutive à l'ingestion de TL 162 augmente significativement la quantité de propionate dans les fèces. Toutefois la souche TL 162 ne s'avérant pas le meilleur candidat pour optimiser la production d'acide propionique dans le côlon (cf critères de sélection *in vitro),* il est vraisemblable que les résultats puissent être améliorés avec une souche sélectionnée selon les critères précités.

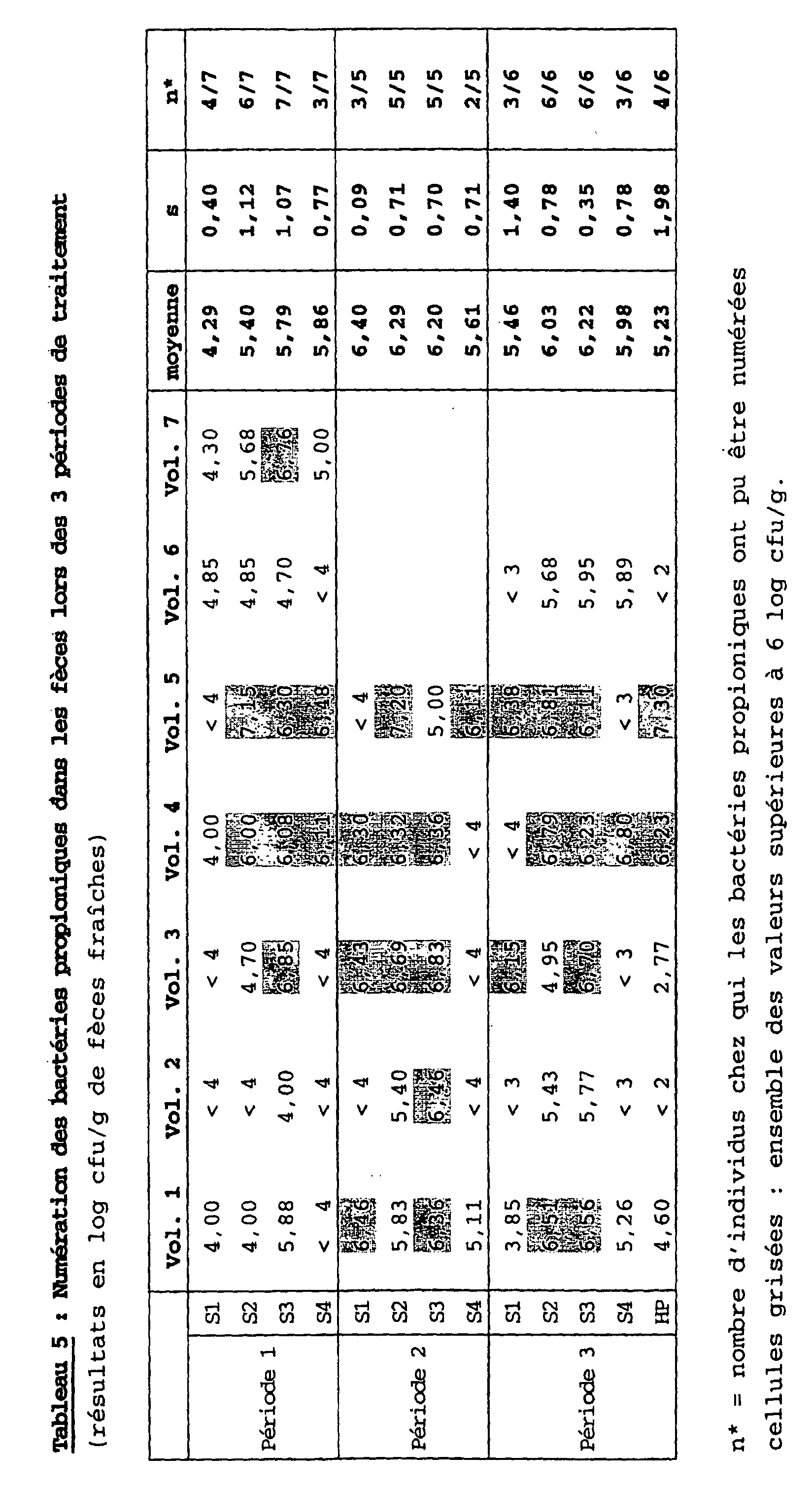

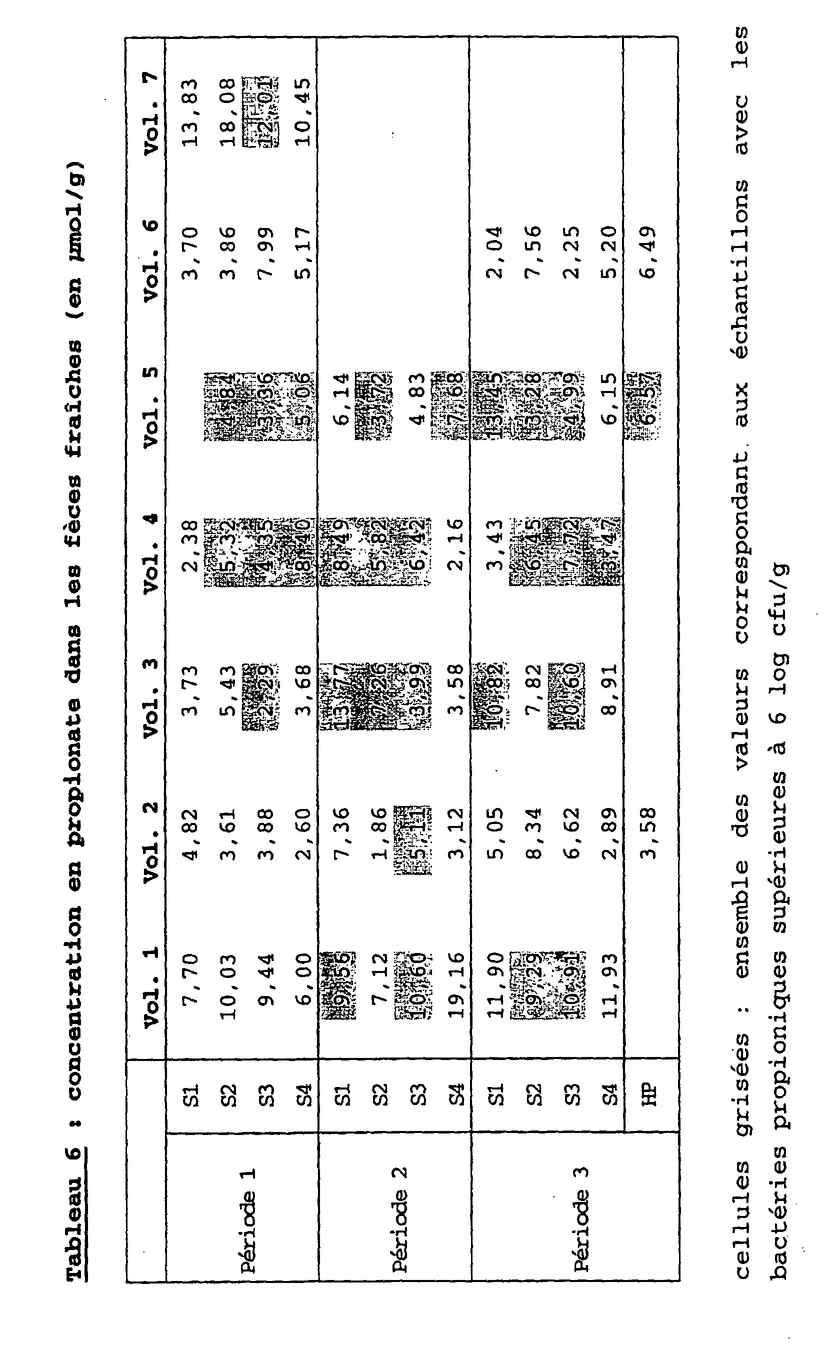

Compte tenu de ce qui précède et conformément à une caractéristique préférentielle de l'invention, les bactéries propioniques utilisées sont choisies parmi les souches produisant de l'acide propionique en quantité physiologiquement significative et en particulier parmi les souches produisant au moins 2 g/l d'acide propionique et/ou de propionates et, de préférence, plus de 4 g/l d'acide propionique et/ou de propionates après avoir été cultivées à 30°C, dans du milieu YEL renfermant environ 11,4 g/l de lactate pendant 2 à 3 jours, puis diluées au 1/10^{ème} dans un milieu YEL contenant 0,6 % de bile de boeuf, incubées à 37°C pendant 90 min, centrifugées, reprises dans du milieu YEL et remises à incuber à 37°C pendant 24 heures.

Un autre critère de sélection pouvant être pris en compte conformément à l'invention correspond aux propriétés d'adhésion des souches sur les côlonocytes : des souches douées de bonnes propriétés d'adhésion présentent en effet l'avantage de demeurer plus longtemps dans le côlon, ce qui leur laisse plus de temps pour synthétiser l'acide propionique ; de plus, les souches qui se fixent, peuvent prendre la place des agents pathogènes.

Il est à noter que pour obtenir l'effet recherché, il n'est pas envisageable de faire absorber l'acide propionique lui-même vu que du fait de la chaîne métabolique humaine il ne pourrait pas parvenir au côlon et que, de plus, il a été montré qu'il est nocif à haute dose pour l'estomac.

Parmi les effets bénéfiques attribués aux acides gras à chaîne courte synthétisés au niveau du côlon et en particulier à l'acide acétique et surtout à l'acide propionique, on peut noter leur rôle au niveau de l'assimilation des principaux minéraux et notamment du calcium, du fer, du zinc ou encore du magnésium ; on a en effet pu établir que l'acide propionique et, dans une moindre mesure, l'acide acétique peut favoriser l'absorption colique de ces minéraux et l'utilisation par l'organisme de la fraction absorbée.

Il s'agit là d'un effet particulièrement intéressant vu que l'assimilation des minéraux s'accompagne d'effets fonctionnels tels qu'à titre d'exemple la correction de l'anémie pour le fer ou la minéralisation osseuse pour le calcium.

Les études expérimentales et cliniques, qui ont été effectuées, fournissent un faisceau d'arguments concordants à l'appui d'un effet favorable de l'acide propionique et des propionates et, dans une moindre mesure, de l'acide acétique et des acétates sur le métabolisme de ces minéraux ; cet effet est vraisemblablement plus important lorsque les conditions de digestion sont mauvaises, ce qui conduit à amener une quantité importante de minéraux non absorbés par l'intestin grêle au niveau colique, et lorsque les besoins sont élevés.

L'existence de relations entre les acides gras à chaîne courte et le métabolisme des minéraux a notamment été suggérée par des études ayant utilisé des fibres solubles. On a ainsi pu établir que les polysaccharides ou oligosaccharides non digérés par les enzymes digestives et qui sont donc fermentés en acides gras à chaîne courte (notamment en propionates) par la flore colique augmentent l'absorption des minéraux tels que le calcium, le fer ou le zinc et que cette augmentation est d'autant plus nette que les conditions sont pathologiques (carences, gastrectomie ...).

Des études de perfusion colique et a contrario l'absence d'effet chez des sujets côlectomisés a permis de confirmer la localisation du site d'action au niveau colique.

Il a en outre été constaté que ces actions s'accompagnent d'une baisse du pH et d'une synthèse d'acides gras à chaîne courte ; ceci suggère l'intervention de ces acides par le biais d'une fermentation, ce d'autant plus que l'on a vérifié que les fibres insolubles, non fermentescibles, n'ont pas d'effet. De plus, il a été établi que le cæcum est hypertrophié et que le débit sanguin colique augmente, ce qui témoigne d'un effet trophique.

Les études cliniques effectuées sur ce sujet sont peu nombreuses, mais permettent de confirmer que les effets des fibres solubles sont obtenus par fermentation colique et de montrer directement l'effet des acides gras à chaîne courte sur l'absorption des minéraux.

Parmi ces études, on peut mentionner la publication « *Trinidad TP, Wolever TMS, Thompson LU, Effect of acetate and propionate on calcium absorption from the rectum and distal côlon of humans*. *Am J Clin Nutr 1996, 63*/ *574-578* ». qui rend compte d'essais dans lesquels on a perfusé directement le côlon distal de sujets sains avec de l'acide acétique, de l'acide propionique ou leur association à concentration physiologique ; on a ainsi établi que la disparition du calcium de la lumière colique est augmentée par les deux acides gras à chaîne courte, mais de façon significativement plus importante par l'acide propionique ; cette étude a également montré un effet dose à l'appui d'un système d'absorption non saturable. Les auteurs ont suggéré que la plus grande lipophilie de l'acide propionique comparé à l'acide acétique pourrait favoriser son absorption et la libération dans le côlonocyte de protons dont le passage dans la lumière digestive favoriserait l'absorption du calcium.

Compte tenu de ce qui précède, l'invention concerne également l'utilisation de bactéries propioniques sélectionnées en fonction de leur caractère peu autolytique et de leur aptitude à résister aux sels biliaires pour l'obtention d'une composition d'alimentation courante ou d'une composition diététique ou médicamenteuse absorbable par l'homme ou l'animal, élaborée pour que les bactéries soient protégées au moins partiellement vis-à-vis de l'acidité gastrique, renfermant au moins 10⁶ cellules/gramme de ces bactéries, susceptible de favoriser l'assimilation des principaux minéraux, en particulier du calcium et/ou du fer et/ou du zinc et/ou du magnésium au niveau du côlon.

Selon une variante de l'invention, il est également proposé d'appliquer cette utilisation à l'obtention d'une composition ayant des propriétés antifongiques au niveau du côlon et, en particulier, susceptible d'y réduire le développement de mycodermes pathogènes du type candida/muguet.

Cette utilisation conduit, en fait à tirer profit des excellentes propriétés antifongiques de l'acide propionique notamment pour le traitement des candidoses dues aux antibiotiques.

Il est à noter que la composition utilisée conformément à l'invention peut, le cas échéant, renfermer des bactéries autres, en particulier des bactéries lactiques et/ou des bactéries bifides susceptibles d'agir en synergie avec les bactéries propioniques de façon à augmenter les effets susmentionnés en leur fournissant du lactate en tant que substrat fermentescible.

La composition utilisée conformément à l'invention peut être constituée par une préparation sèche ou hydratée se présentant sous forme de fractions individuelles d'environ 100 mg à 1 g, de préférence de 200 à 500 mg, renfermant de préférence au moins 10⁸ cellules ; elle peut en particulier avantageusement se présenter sous la forme de gélules ou de capsules gastrorésistantes.

Selon une autre caractéristique de l'invention, cette composition peut également être constituée par une préparation élaborée, les bactéries propioniques étant ajoutées ou associées à un substrat fermentescible notamment à des fibres alimentaires ou encore ajoutées ou incorporées dans des aliments liquides, pâteux ou solides.

Dans une telle préparation, les bactéries propioniques peuvent jouer un double rôle, à savoir technologique dans un premier temps à travers la fermentation d'aliments et fonctionnel dans un second temps, puisqu'une fois ingérées, elles sont capables d'atteindre le côlon et d'y jouer le rôle probiotique susmentionné, en particulier au niveau de l'optimisation de la synthèse d'acide propionique et de l'optimisation de l'assimilation des minéraux.

## Revendications

1. Utilisation de bactéries propioniques appartenant à des souches peu autolytiques et sélectionnées pour leur aptitude à produire au moins 2 g/l d'acide propionique et/ou de propionates et, de préférence, plus de 4 g/l d'acide propionique et/ou de propionates après avoir été cultivées à 30°C, dans un milieu YEL renfermant environ 11,4 g/l de lactate pendant 2 à 3 jours, puis diluées au 1/10^{ème} dans ce milieu YEL additionné de 0,6 % de bile de boeuf, incubées à 37°C pendant 90 minutes, centrifugées, reprises dans du milieu YEL et remises à incuber à 37°C pendant 24 heures, pour l'obtention d'une composition d'alimentation courante ou d'une composition diététique ou médicamenteuse absorbable par l'homme ou l'animal, élaborée pour que les bactéries soient protégées au moins partiellement vis-à-vis de l'acidité gastrique, renfermant au moins 10⁶ cellules par gramme de ces bactéries, susceptible de stimuler et d'augmenter de façon significative la synthèse d'acide propionique et/ou de propionates et le cas échéant d'acide acétique et/ou d'acétates au niveau du côlon par fermentation bactérienne anaérobie.

2. Utilisation selon la revendication 1 pour l'obtention d'une composition susceptible de favoriser l'assimilation des principaux minéraux, en particulier du calcium et/ou du fer et/ou du zinc et/ou du magnésium au niveau du côlon.

3. Utilisation selon la revendication 1,
pour l'obtention d'une composition ayant des propriétés antifongiques au niveau du côlon et, en particulier, susceptible de réduire le développement de mycodermes pathogènes du type candida/muguet.

4. Utilisation selon l'une quelconque des revendications 1 à 3,
**caractérisée en ce que**
les bactéries propioniques appartiennent à des souches douées de propriétés d'adhésion sur les côlonocytes.

5. Utilisation selon l'une quelconque des revendications 1 à 4,
**caractérisée en ce que**
la composition est constituée par une préparation sèche ou hydratée se présentant sous forme de fractions individuelles d'environ 100 mg à 1 g de préférence de 200 à 500 mg, renfermant de préférence au moins 10⁸ cellules.

6. Utilisation selon la revendication 5,
**caractérisée en ce que**
la composition se présente sous la forme de gélules ou de capsules gastrorésistantes.

7. Utilisation selon l'une quelconque des revendications 1 à 6,
**caractérisée en ce que**
la composition est constituée par une préparation élaborée, les bactéries propioniques étant ajoutées ou associées à un substrat fermentescible, notamment à des fibres alimentaires.

8. Utilisation selon l'une quelconque des revendications 1 à 6,
**caractérisée en ce que**
la composition est constituée par une préparation élaborée, les bactéries propioniques étant ajoutées ou incorporées dans des aliments tels que des aliments liquides, pâteux ou solides.

9. Utilisation selon l'une quelconque des revendications 1 à 7,
**caractérisée en ce que**
la composition renferme des bactéries lactiques et/ou des bactéries bifides.

## Claims

1. Use of propioni bacteria belonging to non-very autolytic strains selected for their ability to produce at least 2 g/l propionic acid and/or propionates and preferably more than 4 g/l propionic acid and/or propionates after being cultivated at 30°C in a YEL medium containing approximately 11.4 g/l lactate for 2 to 3 days, then 1/10^{th} diluted in this YEL medium with 0.6% oxgall (bovine bile) added, incubated at 37°C for 90 minutes, centrifuged, returned to the YEL medium and again incubated at 37°C for 24 hours, to obtain a common food composition or a dietary composition or a medicinal composition absorbable by man or animal, developed so that the bacteria are protected at least partially in regard to gastric acidity, containing at least 10⁶ cells per gram of these bacteria, likely to stimulate and significantly increase the synthesis of propionic acid and/or propionates and if necessary acetic acid and/or acetates in the colon by anaerobic bacterial fermentation.

2. Use according to claim 1 for obtaining a composition likely to favour the assimilation of mineral substances, in particular calcium and/or iron and/or zinc and/or magnesium in the colon.

3. Use according to claim 1
for obtaining a composition having anti-fungal properties in the colon and, in particular, likely to reduce the development of pathogenic mycoderms of the candida/thrush type.

4. Use according to any of claims 1 to 3,
**characterised in that**
the propioni bacteria belong to strains with adhesion properties on the colonocytes.

5. Use according to any of claims 1 to 4,
**characterised in that**
the composition is composed of a dry or hydrated preparation being in the form of individual fractions of approximately 100 mg to 1 g, preferably 200 to 500 mg, including preferably at least 10⁶ cells.

6. Use according to claim 5,
**characterised in that**
the composition is in the form of gelatine capsules or gastro-resistant capsules.

7. Use according to any of claims 1 to 6,
**characterised in that**
the composition is composed of a developed preparation, the propioni bacteria being added to or combined with a fermentable substrate, particularly dietary fibre.

8. Use according to any of claims 1 to 6,
**characterised in that**
the composition is composed of a developed preparation, the propioni bacteria being added or incorporated in food such as liquid, paste or solid food.

9. Use according to any of claims 1 to 7,
**characterised in that**
the composition includes lactic bacteria and/or bifid bacteria.

## Patentansprüche

1. Verwendung von Propionsäure-Bakterien, die zu schwach autolytischen Stämmen gehören und ausgewählt sind nach ihrer Fähigkeit zur Produktion von zumindest 2 g/l Propionsäure und/oder Propionaten und vorzugsweise von mehr als 4 g/l Propionsäure und/oder Propionaten nach einer Kultivierung bei 30° C in einem YEL-Medium unter Einschluß von etwa 11,4 g/l Laktat über 2 bis 3 Tage, danach Verdünnung auf 1/10 in dem mit 0,6 % Rindergalle angereicherten YEL-Medium, Inkubation bei 37° C über 90 Minuten, Zentrifugierung, Wiederaufnahme im YEL-Medium und Wiederaufnahme der Inkubation bei 37° C über 24 Stunden, zum Erhalt einer von Mensch oder Tier absorbierbaren Zusammensetzung einer täglichen Nahrung oder einer diätetischen oder medikamentösen Zusammensetzung, in einer solchen Ausarbeitung, daß die Bakterien zumindest teilweise gegenüber der Gastroazidität geschützt sind, unter Einschluß von zumindest 10⁶ Zellen pro Gramm der Bakterien, mit der Fähigkeit, die Synthese von Propionsäure und/oder Propionaten und ggf. von Essigsäure und/oder Azetaten im Bereich des Kolons durch anaerobe Bakterienfermentation in signifikanter Weise zu stimulieren und zu erhöhen.

2. Verwendung nach Anspruch 1, zum Erhalt einer Zusammensetzung, die geeignet ist, die Assimilierung der wesentlichen Mineralien, insbesondere von Kalzium und/oder Eisen und/oder Zink und/oder Magnesium, im Bereich des Kolons zu fördern.

3. Verwendung nach Anspruch 1, zum Erhalt einer Zusammensetzung, die fungizide Eigenschaften im Bereich des Kolons besitzt und insbesondere geeignet ist, die Entwicklung von pathogenen Mykodermen des Typs Candida/Soor zu reduzieren.

4. Verwendung nach einem der Ansprüche 1 bis 3, **dadurch gekennzeichnet, daß** die Propionsäurebakterien zu mit Haftungseigenschaften an den Kolonozyten ausgestatteten Stämmen gehören.

5. Verwendung nach einem der Ansprüche 1 bis 4, **dadurch gekennzeichnet, daß** die Zusammensetzung von einer trockenen oder wasserhaltigen Zubereitung in Form einzelner Fraktionen von etwa 100 mg bis 1 g, vorzugsweise von 200 bis 500 mg, unter Einschluß von vorzugsweise zumindest 10⁸ Zellen gebildet ist.

6. Verwendung nach Anspruch 5, **dadurch gekennzeichnet, daß** die Zusammensetzung die Form von gastroresistenten Gelkörpern oder Kapseln aufweist.

7. Verwendung nach einem der Ansprüche 1 bis 6, **dadurch gekennzeichnet, daß** die Zusammensetzung von einer Zubereitungsausarbeitung gebildet ist, bei der die Propionsäurebakterien einem fermentierungsfähigem Substrat, insbesondere Nahrungsmittelfasern, hinzugefügt oder damit verbunden sind.

8. Verwendung nach einem der Ansprüche 1 bis 6, **dadurch gekennzeichnet, daß** die Zusammensetzung von einer Zubereitungsausarbeitung gebildet ist, bei der die Propionsäurebakterien Nahrungsmitteln wie flüssigen, pastösen oder festen Nahrungsmitteln zugegeben oder in diese eingearbeitet sind.

9. Verfahren nach einem der Ansprüche 1 bis 7, **dadurch gekennzeichnet, daß** die Zusammensetzung Milchsäurebakterien und/oder Bifidusbakterien umfaßt.
